## Europäisches Patentamt

## European Patent Office

## Office européen des brevets

(19)

(11) Veröffentlichungsnummer: **0 192 833**

**A2**

(12) **EUROPÄISCHE PATENTANMELDUNG**

(21) Anmeldenummer: 85115446.8

(22) Anmeldetag: 05.12.85

(51) Int. Cl.⁴: **A 61 B 17/22**

(30) Priorität: 22.02.85 DE 3506249

(43) Veröffentlichungstag der Anmeldung:
03.09.86 Patentblatt 86/36

(84) Benannte Vertragsstaaten:
AT BE CH DE FR GB IT LI LU NL SE

(71) Anmelder: **Messerschmitt-Bölkow-Blohm Gesellschaft
mit beschränkter Haftung
Robert-Koch-Strasse
D-8012 Ottobrunn(DE)**

(72) Erfinder: **Wondrazek, Fritz, Dr.
Riegelstrasse 27
D-8068 Pfaffenhofen(DE)**

(72) Erfinder: **Hahn, Andreas
Ringbergstrasse 19
D-8029 Sauerlach(DE)**

(72) Erfinder: **Hessel, Stefan, Dr.
Widenmayerstrasse 49
D-8000 München 22(DE)**

(72) Erfinder: **Frank, Frank, Dr.
Sahrreiterweg 13
D-8017 Ebersberg(DE)**

(54) Verfahren und Vorrichtung zur Zertrümmerung eines festen Körpers.

(57) Zur Zertrümmerung eines festen Körpers (10), welcher von einem Fluid (14) umgeben ist, insbesondere zur Zertrümmerung von Konkrementen in Lebewesen, mittels eines über einen Lichtleiter (3) geführten Lichtimpulses, wird der aus dem Lichtleiter austretende Lichtimpuls derart fokussiert, daß dessen Energiedichte den Breakdown-Schwellwert des Fluids (14) übersteigt und die dabei entstehende akustische Stoßwelle auf den festen Körper (10) gerichtet.

FIG. 1

EP 0 192 833 A2

21.02.05, 02.83 0192833
ZTT8 Mn/bk
9698

Verfahren und Vorrichtung zur Zertrümmerung eines
festen Körpers

Die Erfindung betrifft ein Verfahren und eine Vorrichtung zur Zertrümmerung eines festen Körpers, welcher
von einem Fluid umgeben ist, insbesondere zur Zertrümmerung von Konkrementen in Lebewesen, mittels eines
über einen Lichtleiter geführten Lichtimpulses

Verfahren und Vorrichtungen dieser Art sind in
"Urologe" (1984) 23, S. 181 - 184 beschrieben. In
diesem Artikel werden mehrere Methoden zur Zerstörung
von Harnsteinen durch Laserstrahlung abgehandelt, wobei
eine dieser Methoden darin besteht, daß ein Harnstein
durch Bestrahlung mit Einzelimpulsen von 24 mJ bei 200
µs bis 20 J bei 10 ms bestrahlt wurden. Dabei wurden
nach 1 bis 3 Impulsen regelmäßig Absprengungen von
Teilchen des Steines beobachtet. In keinem Fall konnte
eine vollständige Zerstörung des Steines erreicht
werden. Eine Schockwelle, die zur Zerstörung der gesamten Kristallstruktur führen sollte, konnte mit dem gepulsten Laser nicht erzeugt werden.

Bei einer anderen Methode wurde mit Hilfe eines gepulsten Lasers (Q-switched, Leistung 13,5 mJ bei 12 ns)
ein Laserstrahl auf ein Ankopplungsmedium (Aluminiumfilm und Glasscheibe) geleitet und somit die Lichtenergie durch eine optomechanische Ankopplung in mechanische Energie verwandelt. Bei direktem Kontakt mit dem
Stein führte der Laserimpuls zum Zerspringen des Steines. Bei diesem Verfahren wird allerdings die Energie
ungerichtet abgegeben, wodurch außer dem Stein auch jedesmal das Ankopplungsmedium und der Lichtleiter zerstört wurden.

0192833

21.02.85, 0391A
ZTT8 Mn/bk
9698

In dem oben erwähnten Artikel wird weiterhin die Vermutung ausgesprochen, daß eine Stoßwelle bei ausreichender Leistungsdichte des Laserimpulses auch direkt in dem, den Stein umgebenden Wasser erzeugt werden könnte. Hierbei wird auf ein ähnliches Prinzip bei der extrakorporalen Stoßwellenlithotrypsie verwiesen, bei dem eine hochgespannte Funkenentladung zur explosionsartigen Verdampfung von Wasser führt. Die dabei frei werdende Energie wird dabei durch einen ellipsoidförmigen Stoßwellenreflektor von außen durch den lebenden Körper auf den zu zertrümmernden Harnstein fokussiert.

Insgesamt wird eine in vivo Anwendung von direkter Laserstrahlung zur Zertrümmerung von Harnsteinen aufgrund thermischer oder mechanischer Schäden des umgebenden lebenden Gewebes noch nicht als sinnvoll angesehen. Hier setzt nun die Erfindung ein, der die Aufgabe zugrundeliegt, ein Verfahren und eine Vorrichtung zur Zertrümmerung von festen Körpern mittels eines über einen Lichtleiter geführten Lichtimpulses zu schaffen, welche an schwer zugänglichen Stellen anwendbar sind und somit insbesondere zur Zertrümmerung von Konkrementen in Lebewesen geeignet ist.

Die Erfindung wird durch ein Verfahren sowie durch eine Vorrichtung nach den kennzeichnenden Merkmalen der Ansprüche 1 und 5 gelöst. Die Erfindung macht sich die Erkenntnis zunutze, daß laserinduzierte Stoßwellen durch den sog. "Breakdown-Effekt" erzeugt werden können (Dissertation von Dipl.-Phys. Jürgen Munschau "Theoretische und experimentelle Untersuchungen zur Erzeugung, Ausbreitung und Anwendung laserinduzierter Stoßwellen", TU Berlin, Berlin 1981). Mit Breakdown wird dabei die starke Ionisation eines Mediums im Brennpunkt eines fokussierten Laserstrahles bezeichnet. Die dabei im

0192833
21.02.85, 0391A
ZTT8 Mn/bk
9698

Fokalbereich umgesetzte Energie steigt durch Kaskadenionisation etwa exponentiell mit der Zeit an. Infolge des Expansionsbestrebens des heißen Plasmas
wächst der Druck, den das Plasma auf seine Umgebung
ausübt, also ordentlich stark an. Diese Expansion erfolgt so rapide, daß auf das umgebende Medium ein Stoß
ausgeübt wird, ähnlich wie bei einer Explosion. In
größerer Entfernung von dem Plasmabereich geht die
Stoßwelle, unabhängig von der ursprünglichen Form des
Plasmabereichs, in eine Kugelwelle über. Der Breakdown-Schwellwert, also der Wert der Strahlungsintensität, bei der die Plasmasbildung einsetzt, ist von dem
jeweiligen Fluid abhängig und beträgt beispielsweise in
Luft unter Atmosphärendruck ca. $2 \cdot 10^{14}$ W/m$^2$ und
bei destilliertem Wasser ca. $6,4 \cdot 10^{13}$ W/m$^2$. Nähere Angaben zur Erzeugung laserinduzierter Stoßwellen,
insbesondere zu den verwendeten Lasergeräten sowie zu
dem Einfluß der Optik, können der obengenannten Dissertation entnommen werden.

Im folgenden wird die Erfindung anhand zweier teilweise
schematisch dargestellter Ausführungsbeispiele näher
beschrieben. Es zeigen:

Fig. 1    eine Einrichtung zur Zerstörung fester Ablagerungen im menschlichen Körper mit einer am Ende
          eines Lichtleiters angeordneten Einrichtung zur
          Erzeugung von Stoßwellen;

Fig. 2    eine weitere Ausführungsform eines am Ende
          eines Lichtleiters angeordnete Einrichtung zur
          Erzeugung von Stoßwellen.

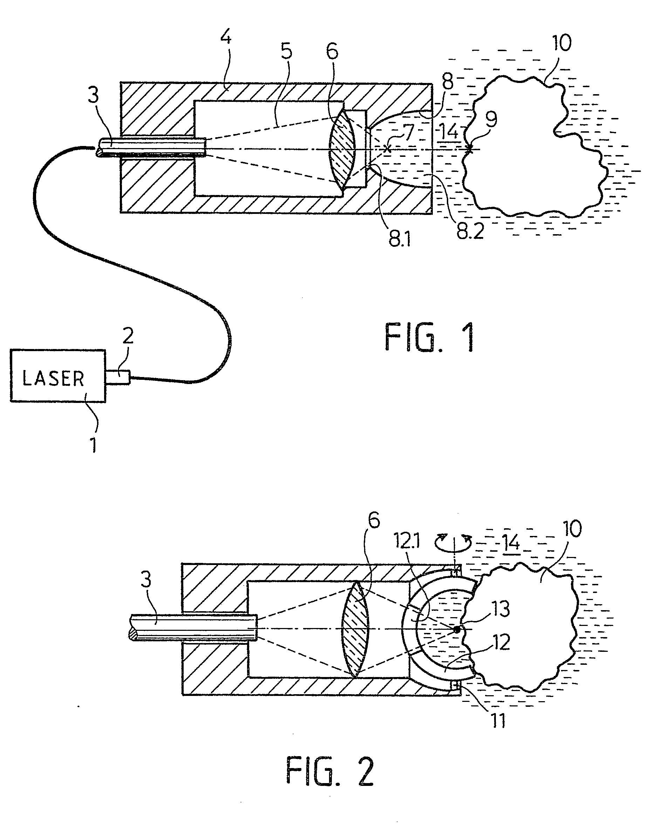

Die in Fig. 1 dargestellte Ausführungsform weist einen
Q-switched-Laser 1 auf, der geeignet ist, Lichtimpulse

0192833
21.02.85, 0391A
ZTT8 Mn/bk
9698

mit einer Pulsdauer von etwa 10 ns und einer Energie von 50 mJ auszusenden. Unter Licht wird dabei der gesamte optische Spektralbereich der elektromagnetischen Wellen verstanden, welcher vom Infraroten bis zum Ultravioletten reicht. Die Laserstrahlung aus dem Laser 1 wird über eine Einkoppeloptik 2 in eine Lichtleitfaser 3 eingekoppelt, deren austrittsseitiges Ende in ein stark vergrößert dargestelltes Gehäuse 4 mündet. Der divergent aus dem Lichtleiter 3 austretende Laserstrahl 5 wird mittels einer Optik 6 auf einen Punkt 7 fokussiert. Dieser Punkt 7 ist gleichzeitig der erste Fokuspunkt eines ellipsoidförmigen Reflektors 8, welcher durch das Gehäuse 4 gebildet wird und eine Lichteintrittsöffnung 8.1 sowie eine Austrittsöffnung 8.2 für die reflektierte Stoßwelle aufweist. Dieser Reflektor 8 hat die Aufgabe, Stoßwellen, welche im Punkt 7 erzeugt werden, durch Reflexion auf den zweiten Fokuspunkt 9 des Reflektors zu fokussieren. Der Reflektor soll so beschaffen sein, daß ein möglichst hoher Anteil der im ersten Fokuspunkt erzeugten Wellenenergie möglichst phasenrichtig in den zweiten Fokuspunkt übertragen wird. Derartige Reflektoren sind beispielsweise aus der DE-OS 23 51 247 oder DE-OS 32 41 026 bekannt.

Der Lichtleiter 3 mit dem relativ schmalen Gehäuse 4 wird zur Zerstörung einer festen Ablagerung entweder durch eine natürliche Körperöffnung oder durch einen geschaffenen schmalen Kanal in die Nähe der Ablagerung 10 so gebracht, daß der zweite Brennpunkt 9 entweder auf der Oberfläche oder knapp innerhalb der Ablagerung 10 liegt. In der Regel wird mit dem Lichtleiter eine nicht dargestellte Kanüle mitgeführt, über die eine Spülflüssigkeit 14 in dem Bereich zwischen dem Reflektor 8 und dem zu zerstörenden Körper 10 gebracht

0192833
21.02.85, 0391A
ZTT8 Mn/bk
9698

wird. In dieser wasserhaltigen Flüssigkeit 14 wird dann mittels eines Laserimpulses der oben erwähnte Breakdown-Effekt erzeugt und die dabei entstehende akustische Stoßwelle über den Reflektor 8 auf den zu zerstörenden Körper ge- richtet. Das Fluid 14, welches ggf. auch gasförmig sein kann, sollte sowohl die gewünschten optischen Eigen- schaften hinsichtlich des Breakdown-Effektes aufweisen, als auch eine möglichst verlustarme Übertragung der entstehenden Stoßwelle erlauben.

Fig. 2 zeigt eine am Ende des Lichtleiters 3 angeordnete Einrichtung zur Erzeugung einer Stoßwelle, bei der sich an die Fokussieroptik 6 ein um eine Achse 11 schwenkbarer Kugelreflektor 12 anschließt, dessen Brennpunkt 13 mit dem Brennpunkt der Optik 6 zusammenfällt. Der Reflektor 12 besteht entweder vollständig oder nur in einem Bereich 12.1 aus einem für die Laserstrahlung transparenten, hochfesten Material, z.B. Glaskeramik. Der äußere Rand des Reflektors dient gleichzeitig als Anschlag für den zu zerstörenden Körper 10 und ist so weit vorgezogen, daß bei Berührung der Körper 10 mit seiner Oberfläche in der Nähe des Brennpunktes 10 liegt. Die dargestellten Ausführungsbeispiele zeigen, daß mit der Einrichtung eine exakte Applikation auf kleinstem Raum direkt am Konkrement möglich ist.

Weitere Ausführungsformen, insbesondere Variationen der Ausgestaltungen des Reflektors für die Fokussierung der akustischen Stoßwellen sind ,abhängig vom jeweiligen Verwendungszweck, möglich.

0192833
21.02.85, 0391A
ZTT8 Mn/bk
9698

Verfahren und Vorrichtung zur Zertrümmerung eines festen Körpers

Patentansprüche

1. Verfahren zur Zertrümmerung eines festen Körpers, welcher von einem Fluid umgeben ist, insbesondere zur Zertrümmerung von Konkrementen in Lebewesen, mittels eines über einen Lichtleiter geführten Lichtimpulses, dadurch g e k e n n z e i c h n e t , daß der aus dem Lichtleiter austretende Lichtimpuls derart fokussiert wird, daß dessen Energiedichte den Breakdown-Schwellwert des Fluids übersteigt und, daß die dabei entstehende akustische Stoßwelle auf den festen Körper gerichtet wird.

2. Verfahren nach Anspruch 1, dadurch g e k e n n z e i c h n e t , daß die akustische Stoßwelle mittels eines Reflektors auf den Festkörper fokussiert wird.

3. Verfahren nach Anspruch 1 oder 2, dadurch g e k e n n z e i c h n e t , daß der Breakdown-Schwellwert des Fluids in einem ersten Brennpunkt eines ellipsoidförmigen Reflektors erreicht wird und der zweite Brennpunkt des Reflektors auf die Oberfläche oder einen Punkt innerhalb des festen Körpers gerichtet wird.

4. Verfahren nach Anspruch 1 oder 2, dadurch g e k e n n z e i c h n e t , daß der Breakdown-Schwellwert des Fluids im Brennpunkt eines

0192833

21.02.85, 0391A
ZTT8 Mn/bk
9698

Kugelreflektors erreicht wird und der feste Körper in unmittelbarer Nähe des Brennpunktes positioniert ist.

5. Vorrichtung zur Durchführung des Verfahrens nach einem der Ansprüche 1 bis 4
g e k e n n z e i c h n e t  durch
a) eine Impulslichtquelle (1),
b) einen mit der Impulslichtquelle verbundenen Lichtleiter (3),
c) eine am austrittsseitigen Ende des Lichtleiters (3) angeordnete optische Fokussiereinrichtung und durch
d) einen Reflektor (8,12)zur Fokussierung von Stoßwellen.

6. Vorrichtung nach Anspruch 5,  dadurch g e k e n n z e i c h n e t ,  daß der Brennpunkt (7,13) der optischen Fokussiereinrichtung (6) innerhalb des Reflektors (8,12) zur Fokussierung von Stoßwellen liegt.

7. Vorrichtung nach Anspruch 5 oder 6,  dadurch g e k e n n z e i c h n e t ,  daß der Brennpunkt (7,13) der optischen Fokussiereinrichtung (6) mit einem Brennpunkt des Reflektors (8,12) zur Fokussierung von Stoßwellen zusammenfällt.

8. Vorrichtung nach einem der Ansprüche 5 bis 7, dadurch  g e k e n n z e i c h n e t ,  daß der Lichtleiter eine Lichtleitfaser (3) ist, dessen austrittsseitiges Ende in ein Gehäuse (4) mündet, welches eine Fokussieroptik (6) sowie einen Reflektor (8) zur Fokussierung von Stoßwellen mit einer Lichteintrittsöffnung (8.1) und einer Stoßwellenaustrittsöffnung (8.2) aufweist.

9. Vorrichtung nach Anspruch 8, dadurch gekennzeichnet , daß zumindest die Lichteintrittsöffnung (12.1) des Reflektors (12) zur Fokussierung von Stoßwellen aus einem optisch transparenten Material besteht und einen Teil des Reflektors bildet.

10. Vorrichtung nach einem der Ansprüche 5 bis 9, dadurch gekennzeichnet , daß der Reflektor (12) zur Fokussierung von Stoßwellen um zumindest eine durch einen Brennpunkt (13) verlaufende Achse (11) schwenkbar ist.

FIG. 1

FIG. 2